# EUROPÄISCHE PATENTSCHRIFT

(11) **EP 0 614 657 B1**
(45) Veröffentlichungstag und Bekanntmachung des Hinweises auf die Patenterteilung: **19.04.1995**
(21) Anmeldenummer: 94100958.1
(22) Anmeldetag: 24.01.1994
(51) Int. Cl.: A61K 7/09

(54) **Mittel zur dauerhaften Verformung von menschlichen Haaren**
Composition for permanent waving of human hair
Composition pour l'ondulation permanente des cheveux humains

(30) Priorität: 17.02.1993 DE 4304828; 05.03.1993 DE 4306915
(43) Veröffentlichungstag der Anmeldung: 14.09.1994
(73) Patentinhaber: GOLDWELL GmbH, D-64297 Darmstadt (DE)
(72) Erfinder: Rose, Burkhard, D-64297 Darmstadt (DE); Tennigkeit, Jürgen, Dr., D-64342 Seeheim (DE)

(56) Entgegenhaltungen:
- EP-A- 0 261 387
- EP-A- 0 362 663
- DE-C- 4 109 365

## Beschreibung

Die vorliegende Erfindung betrifft ein Mittel zum Verformen von menschlichen Haaren, d.h. ein Dauerwellmittel, das eine ausgezeichnete Wellwirkung besitzt, jedoch auf das Haar auch bei mehrfacher Anwendung keinerlei schädigende Wirkung ausübt.

Die Dauerwellung erfordert bekanntlich zwei Behandlungsschritte: Die reduktive Spaltung der Cystin-Disulfidbrücken des Haares durch Einwirkung eines Reduktionsmittels und die anschließende Neutralisierung bzw. Fixierung durch Aufbringung eines Oxidationsmittels, wodurch die Cystin-Disulfidbrücken wiederhergestellt werden.

Das überwiegend eingesetzte Reduktionsmittel ist auch heute noch Thioglykolsäure, insbesondere als Ammoniumsalz, obwohl zahlreiche andere Thio-Verbindungen für diesen Zweck vorgeschlagen wurden, die sich jedoch in der Praxis nicht durchgesetzt haben.

Die Thioglykolat enthaltenden Zusammensetzungen werden üblicherweise bei einem pH-wert zwischen 8 und 10, insbesondere 8,5 bis 9,5, eingesetzt, was bei wiederholter, zeitlich nahe zusammenliegender Anwendung zu Haarschädigungen führen kann.

Man hat bereits versucht, diese Nachteile durch die Schaffung sogenannter "saurer Dauerwellmittel" zu überwinden, deren Anwendungs-pH-Wert bei etwa 6,8 bis 7,8, d.h. um den Neutralpunkt herum, liegt. Der in diesen Zusammensetzungen meistbenutzte reduzierende Wirkstoff ist der Thioglykolsäuremonoglycerinester. Es hat sich jedoch gezeigt, daß diese Substanz bei manchen Benutzerinnen hautreizend, insbesondere sensibilisierend, wirkt, so daß auch diese Lösung nicht optimal ist.

Es wurde nunmehr gefunden, daß diese Probleme dadurch überwunden werden können und Dauerwellmittel, die bei einem pH-Wert wirken, wo keine Haarschädigung auftritt, jedoch gleichwohl eine gute Wellwirkung aufweisen, erhalten werden, wenn man ein Mittel einsetzt, das Thioglykolsäure bzw. ein Salz derselben, vorzugsweise Ammoniumthioglykolat, enthält, und unmittelbar vor der Anwendung durch Vermischen von zwei bis dahin getrennnt gehaltenen Zusammensetzungen hergestellt wird, wobei die eine Zusammensetzung (A) mindestens ein Aminosäurehydrochlorid und mindestens ein Polyol bzw. dessen Methyl- und Ethylether auf wäßriger Grundlage enthält, und einen pH-Wert zwischen 4,5 und 6,5, Vorzugsweise zwischen 5 und 6, insbesondere zwischen 5,4 und 5,6 aufweist, und eine Zusammensetzung (B), die als Alkalisierungsmittel Ammoniumhydrogencarbonat, Ammoniumcarbonat und/oder Ammoniumcarbamat enthält und einen pH-Wert zwischen etwa 8 und 9,5, insbesondere 8,2 bis 8,6, vorzugsweise 8,3 bis 8,4, aufweist, und die dadurch erhaltene, gebrauchsfertige Zusammensetzung einen pH-Wert zwischen 7 und 8, vorzugsweise 7,4 und 7,6, besitzt.

Diese Zusammensetzungen sind stabil und weisen auch nur einen geringen Druckaufbau auf.

Die Zusammensetzung enthält, wie bereits ausgeführt, Thioglykolsäure und/oder deren Ammoniumsalz, jedoch können auch weitere Salze, beispielsweise Natriumthioglykolat oder Aminthioglykolate, verwendet werden. Die Mitverwendung weiterer wellwirksamer Thio-Verbindungen, wie sie an sich bekannt sind, ist möglich, jedoch nicht zwingend erforderlich.

Vorzugsweise werden die Thioglykolsäure bzw. deren Salze in der Zusammensetzung (A) eingesetzt.

Der Anteil an Thioglykolsäure in den erfindungsgemäßen Zusammensetzungen liegt zwischen etwa 4 und etwa 12 Gew.-%, Vorzugsweise etwa 6 bis 9 Gew.-%, jeweils berechnet auf Thioglykolsäure, bezogen auf die Gesamtzusammensetzung des gebrauchsfertigen Mittels (Zusammensetzung A + B).

Der essentielle Bestandteil der Zusammensetzung (A) ist ein Aminosäurehydrochlorid, das in der Mischung von Thioglykolsäure bzw. deren Salzen und Polyol zur pH-Einstellung und damit zur Stabilität des Systems beiträgt.

Bevorzugtes Aminosäurehydrochlorid ist das Cysteinhydrochlorid, das darüber hinaus auch noch eine gewisse Wellwirksamkeit aufweist. Ein weiteres bevorzugtes Aminosäurehydrochlorid ist das Glycinhydrochlorid.

Es können jedoch auch weitere Aminosäurehydrochloride, beispielsweise die des Alanins, Valins, Leucins, Isoleucins, Prolins, Tryptophans, Phenylalanins, Methionins, Serins, Tyrosins, Threonins, Asparagins, Glutamins oder Histidins enthalten sein.

Der Anteil des Aminosäurehydrochlorids wird bestimmt durch die Einstellung des erforderlichen pH-Wertes; der Mindestanteil liegt bei etwa 0,5 Gew.-%, der Höchstanteil bei etwa 5 Gew.-%, bezogen auf reine Aminosäure und die Gesamtzusammensetzung des Mittels (also die Summe der Zusammensetzungen (A) und (B)).

Der dritte essentielle Bestandteil in den die Thioglykolsäure bzw. deren Salze enthaltenden Zusammensetzungen ist ein Polyol bzw. dessen Ether. Als solche werden insbesondere 1,2- oder 1,3-Propandiol, 1,2-, 1,3- oder 1,4-Butandiol sowie deren Methyl- und Ethylether, insbesondere 1-Methoxypropanol(-2), Dipropylenglykolmonomethylether und Glycerin eingesetzt.

Die bevorzugte Menge liegt bei etwa 1 bis 15 Gew.-% der Gesamtzusammensetzung (Zusammensetzungen (A) + (B)), vorzugsweise bei etwa 2 bis 10 Gew.-%.

Die von der Zusammensetzung (A) bis zur Anwendung getrennt gehaltene Zusammensetzung (B) enthält Ammoniumhydrogencarbonat, Ammoniumcarbonat und/oder Ammoniumcarbamat als Alkalisierungsmittel, vorzugsweise in einer Menge zwischen etwa 1 und etwa 10 Gew.-%, bevorzugt 2 bis etwa 7, insbesondere etwa 2,5 bis 6 Gew.-%, berechnet auf die Gesamtzusammensetzung (A) + (B).

Bevorzugt enthält (B) 1-5 Gew.-% Ammoniumhydrogencarbonat, berechnet auf die Gesamtzusammensetzung (A) + (B).

Die erfindungsgemäßen Dauerwellmittel können alle für diesen Zweck vorgeschlagenen und eingesetzten Zusatzstoffe enthalten.

Zur Vermeidung von Wiederholungen wird hierzu auf den einschlägig bekannten Stand der Technik und dessen Zusammenfassungen, beispielsweise in "Ullmann's Encyclopaedia of Industrial Chemistry", Vol.A12 (1986), S.588 bis 591, sowie die Monographie Von K.Schrader, "Grundlagen und Rezepturen der Kosmetika", 2. Auflage (1989, Hüthig Buchverlag), S.823 bis 840, verwiesen.

Die Fixierung der durch Anwendung der erfindungsgemäßen Dauer-well-Zusammensetzungen verformten Haare erfolgt durch die ebenfalls bekannte und übliche Neutralisation mittels Wasserstoffperoxid oder Alkalibromaten.

Die getrennte Unterbringung der beiden Zusammensetzungen gemäß der Erfindung bis zur Anwendung des Mittels geschieht vorzugsweise in den seit langem bekannten Zweikammerbehältern, deren Trennwand bei der Anwendung durch geeignete Mittel zerstört wird.

Solche Zweikammerbehältnisse, die sich insbesondere für die erfindungsgemäßen Zusammensetzungen eignen, sind beispielsweise in den deutschen Offenlegungsschriften 35 28 525, 36 06 003, 38 12 343 und 38 37 595 beschrieben.

Die folgenden Beispiele dienen der Illustration der Erfindung.

### Beispiel 1

| Zusammensetzung A: | |
|---|---|
| Ammoniumthioglykolat, 71%ig | 11,0 (g) |
| Cysteinhydrochlorid · H₂O | 2,5 |
| 1,2-Propandiol | 2,5 |
| Ammoniak, 25%ig | @ pH 5,5 |
| Wasser | @ 27,8 |

| Zusammensetzung B: | |
|---|---|
| Ammoniumhydrogencarbonat | 5,0 (g) |
| Quaternäres Polymer | 0,5 |
| Amphoteres Tensid (Coco-Betain) | 0,8 |
| Diacetin | 0,2 |
| 1,3-Butandiol | 1,0 |
| 1,2-Propandiol | 0,5 |
| Nichtionisches Tensid | 0,8 |
| Farbstoff, Trübungsmittel, Parfum | q.s. |
| Ammoniak, 25%ig | @ pH 8,3 |
| Wasser | @ 72,2 |

Die Zusammensetzungen wurden separat in Portionspackungen zu 72,2 g (Zusammensetzung B) bzw. 27,8 g (Zusammensetzung A) in eine bekannte Zweikammerpackung gefüllt.

### Beispiel 2

| Zusammensetzung A: | |
|---|---|
| Ammoniumthioglykolat | 18,0 (g) |
| Glycinhydrochlorid | 0,7 |
| 1,2-Propandiol oder Glycerin | 2,5 |
| Ammoniak, 25%ig | @ pH 5,5 |
| Wasser | @ 27,8 |

| Zusammensetzung B: | |
|---|---|
| Ammoniumhydrogencarbonat | 2,0 (g) |
| Quaternäres Fettalkylammoniumchlorid | 1,5 |
| Amphoteres Tensid | 0,7 |
| Ethanol | 5,0 |
| Nichtionisches Tensid | 0,8 |
| Diacetin | 0,6 |
| Parfum, Entschäumer, Trübungsmittel | q.s. |
| Ammoniak, 25%ig | @ pH 8,4 |
| Wasser | @ 72,2 |

Die Zusammensetzungen (A) und (B) werden, wie in Beispiel 1 beschrieben, separat in eine Zweikammerdose verpackt.

### Beispiel 3

| Zusammensetzung A: | |
|---|---|
| Ammoniumthioglykolat, 71%ig | 10,5 (g) |
| Cysteinhydrochlorid · H₂O | 2,5 |
| 1,2-Propandiol | 2,5 |
| Ethanol | 2,0 |
| Ammoniak, 25%ig | @ pH 5,6 |
| Wasser | @ 27,8 |

| Zusammensetzung B: | |
|---|---|
| Ammoniumhydrogencarbonat | 5,0 (g) |
| Harnstoff | 1,0 |
| Quaternäres Polymer | 1,0 |
| Amphoteres Tensid | 0,8 |
| Ethanol | 0,5 |
| Isopropylalkohol | 1,0 |
| Diacetin | 0,3 |
| Nichtionischer Emulgator | 0,8 |
| Farbstoff, Trübungsmittel, Parfum | q.s. |
| Ammoniak, 25%ig | @ pH 8,4 |
| Wasser | @ 72,2 |

27,8 g der Zusammensetzung (A)und 72,2 g der Zusammensetzung (B) werden analog Beispiel 1 in eine Zweikammerdose verpackt.

### Beispiel 4

| Zusammensetzung A: | |
|---|---|
| Ammoniumthioglykolat, 71%ig | 18,2 (g) |
| Cysteinhydrochlorid · H₂O | 3,0 |
| 1,2-Propandiol | 2,5 |
| Ammoniak, 25%ig | @ pH 5,5 |
| Wasser | @ 27,8 |

| Zusammensetzung B: | |
|---|---|
| Ammoniumcarbonat | 2,9 (g) |
| Quaternäres Polymer | 0,5 |
| Amphotere Tenside (Coco-Betain) | 0,6 |
| Diacetin | 0,2 |
| Ethanol | 5,0 |
| 1,3-Butandiol | 1,0 |
| 1,2-Propandiol | 0,5 |
| Nichtionisches Tensid | 0,8 |
| Farbstoff, Trübungsmittel, Parfum | q.s. |
| Wasser | @ 72,2 |
| (pH: ≈ 8,75) | |

Die Zusammensetzungen wurden separat in Portionspackungen zu 65 g (Zusammensetzung B) bzw. 25 g (Zusammensetzung A) in eine bekannte Zweikammerpackung gefüllt.

### Beispiel 5

| Zusammensetzung A: | |
|---|---|
| Ammoniumthioglykolat | 18,3 (g) |
| Cysteinhydrochlorid | 3,0 |
| 1,2-Propandiol oder Glycerin | 2,5 |
| Ammoniak, 25%ig | @ pH 5,5 |
| Wasser | @ 27,8 |

| Zusammensetzung B: | |
|---|---|
| Ammoniumcarbamat | 0,9 (g) |
| Quaternäres Polymeres | 0,4 |
| Amphotere Tenside (Coco-Betain) | 0,6 |
| Ethanol | 5,0 |
| Nichtionisches Tensid | 0,8 |
| Diacetin | 0,3 |
| Parfum, Entschäumer, Trübungsmittel | q.s. |
| Wasser | @ 72,2 |
| (pH-Wert: 9,18) | |

Die Zusammensetzungen (A) und (B) werden, wie in Beispiel 1 beschrieben, separat in eine Zweikammerdose verpackt.

Beim Vermischen einer der bis zur Anwendung getrennt gehaltenen Zusammensetzungen 1A bis 5A mit einer der Zusammensetzungen 1B bis 5B werden gebrauchsfertige Mischungen mit einem nur schwach alkalischen pH-Wert von etwa 7,4 bis etwa 7,6 erhalten, die beim Aufbringen auf das Haar und anschließender Neutralisierung bzw. Fixierung mit verdünntem Wasserstoffperoxid eine ausgezeichnete Wellwirkung ergeben und gleichwohl das Haar auch bei mehrfach kurz aufeinanderfolgender Behandlung nicht schädigen.

### Beispiel 6

| Zusammensetzung A | |
|---|---|
| 1,2-Propandiol | 2,5 (g) |
| Cysteinhydrochlorid | 2,5 |
| Ammoniak, 25%ig | @ pH 5,5 |
| Wasser | @ 27,8 |

| Zusammensetzung B | |
|---|---|
| Ammoniumhydrogencarbonat | 5,0 (g) |
| Quaternäres Polymeres | 0,5 |
| Amphoteres Tensid (Coco-Betain) | 0,8 |
| Nichtionisches Tensid | 0,8 |
| Diacetin | 0,2 |
| 1,3-Butandiol | 1,0 |
| 1,2-Propandiol | 0,5 |
| Ammoniumthioglykolat, 71%ig | 11,0 |
| Ammonium-2-thiolactat, 50%ig | 2,0 |
| Farbstoff, Trübungsmittel, Parfum | q.s. |
| Ammoniak, 25%ig | @ pH 8,0 |
| Wasser | @ 72,2 |

25 g der Zusammensetzung (A) und 65 g der Zusammensetzung (B) wurden, wie in den vorhergehenden Beispielen beschrieben, in eine Zweikammerdose verpackt. Bei vermischen der Zusammensetzungen (A) und (B) wurde eine gebrauchsfertige Dauerwell-Lösung mit einem pH-Wert von 7,7 erhalten, die beim Aufbringen und Einwirken auf das Haar und anschließender Neutralisierung bzw. Fixierung mit verdünnter H₂O₂-Lösung eine ausgezeichnete Dauerwelle ergab, wobei auch nach mehrfacher Anwendung keine Schädigung des Haares auftrat.

## Patentansprüche

1. Mittel zur dauerhaften Verformung von menschlichen Haaren, das Thioglykolsäure und/oder ein Salz derselben enthält und aus zwei bis zur Anwendung voneinander getrennt gehaltenen Zusammensetzungen besteht, dadurch gekennzeichnet, daß die eine Zusammensetzung (A) mindestens ein Aminosäurechlorid und mindestens ein Polyol bzw. dessen Methyl- oder Ethylether in wäßriger Lösung enthält, und einen pH-Wert zwischen etwa 4,5 und 6,5 aufweist, und die zweite, davon getrennt gehaltene Zusammensetzung (B) Ammoniumhydrogencarbonat, Ammoniumcarbonat und/oder Ammoniumcarbamat enthält, wobei der pH-wert zwischen etwa 8 und 9,5 liegt, und beide Zusammensetzungen unmittelbar vor der Anwendung auf menschliches Haar vermischt werden, und die dadurch hergestellte gebrauchsfertige Zusammensetzung (AB) einen pH-Wert zwischen 7 und 8 aufweist.

2. Mittel zur dauerhaften verformung von menschlichen Haaren nach Anspruch 1, dadurch gekennzeichnet, daß die Zusammensetzung (A) Thioglykolsäure und/oder ein Salz derselben in Kombination mit Aminosäurehydrochloriden und mindestens einem Polyol bzw. dessen Methyl- oder Ethylethern in wäßriger Lösung enthält.

3. Mittel nach Anspruch 1 oder 2, dadurch gekennzeichnet, daß die Zusammensetzung (A) einen pH-Wert zwischen 5,0 und 6,0 aufweist.

4. Mittel nach Anspruch 1 oder 2, dadurch gekennzeichnet, daß die Zusammensetzung (B) einen pH-Wert zwischen 8,1 und 8,6 aufweist.

5. Mittel nach einem der vorhergehenden Ansprüche, dadurch gekennzeichnet, daß die nach dem Vermischen der Zusammensetzungen (A) und (B) erhaltene, auf das Haar aufzubringende gebrauchsfertige Zusammensetzung (AB) einen pH-Wert zwischen 7,3 und 7,7 aufweist.

6. Mittel nach einem der vorhergehenden Ansprüche, dadurch gekennzeichnet, daß es als Aminosäurehydrochlorid Cysteinhydrochlorid enthält.

7. Mittel nach einem oder mehreren der vorhergehenden Ansprüche, dadurch gekennzeichnet, daß es als Aminosäurehydrochlorid Glycinhydrochlorid enthält.

8. Mittel nach einem oder mehreren der vorhergehenden Ansprüche, dadurch gekennzeichnet, daß die Zusammensetzung (A) 1 bis 15 Gewichtsprozent eines Polyols bzw. eines Methyl- oder Ethylethers desselben enthält.

9. Mittel nach Anspruch 8, dadurch gekennzeichnet, daß die Zusammensetzung (A) 1,2-Propandiol und/oder Glycerin enthält.

10. Mittel nach Anspruch 8, dadurch gekennzeichnet, daß die Zusammensetzung (A) 1-Methoxypropanol(-2) und/oder Dipropylenglykolmonomethylether enthält.

11. Mittel nach einem oder mehreren der vorhergehenden Ansprüche, dadurch gekennzeichnet, daß es 1 bis 5 Gew.-% Ammoniumhydrogencarbonat enthält.

## Claims

1. Composition for permanent waving of human hair, containing thioglycolic acid or a salt thereof and comprising two compositions which are kept separate until application, whereby a Composition (A) contains at least one amino acid hydrochloride, and at least one polyol or methyl or ethyl ethers thereof in an aqueous solution, having a pH-value of about 4.5 to 6.5, and a second Composition (B), being kept separate from Composition (A), contains ammonium carbonate, ammonium hydrogen carbonate and (or) ammonium carbamate, wherein the pH-value is about between 8 and 9.5, whereof both compositions are mixed before application onto human hair, and the ready-to-use Composition (AB) produced thereby has a pH-value between 7 and 8.

2. Composition for permanent waving of human hair according to claim 1, wherein the Composition (A) contains thioglycolic acid and (or) a salt thereof in combination with amino acid hydrochlorides and at least one polyol or methyl or ethyl ether thereof in aqueous solution.

3. Composition according to claim 1 or 2, characterized in that Composition (A) has a pH-value between 5.0 and 6.0.

4. Composition according to claim 1 or 2, characterized in that Composition (B) has a pH-value between 8.5 and 9.2.

5. Composition according to one of the preceding claims, characterized in that Composition (AB), ready-to-use for application onto the hair, obtained by admixture of Compositions (A) and (B), has a pH-value between 7.3 and 7.7.

6. Composition according to one of the preceding claims, characterized in that it contains cysteine hydrochloride as the amino acid hydrochloride.

7. Composition according to one or more of the preceding claims, characterized in that it contains glycine hydrochloride as the amino acid hydrochloride.

8. Composition according to one or more of the preceding claims, characterized in that Composition (A) comprises 1 to 15% by weight of a polyol or a methyl or ethyl ether thereof.

9. Composition according to claim 8, characterized in that Composition (A) comprises 1,2-propanediol and (or) glycerol.

10. Composition according to claim 8, characterized in that Composition (A) comprises 1-methoxypropanol(-2) and (or) dipropyleneglycol monomethylether.

11. Composition according to one or more of the preceding claims, characterized in that it comprises 1 to 5% by weight ammonium hydrogen carbonate.

## Revendications

1. Produit pour la déformation durable des cheveux humains, qui contient de l'acide thioglycolique et/ou un de ses sels, et qui est constitué par deux compositions conservées séparément jusqu'à l'utilisation, caractérisé en ce que l'une des compositions (A) contient au moins un chlorure d'aminoacide et au moins un polyol ou son éther de méthyle ou d'éthyle en solution aqueuse et présente un pH entre environ 4,5 et 6,5, la seconde composition (B) conservée séparée de la première contient un hydrogénocarbonate d'ammonium, un carbonate d'ammonium et/ou un carbamate d'ammonium, le pH étant entre 8 et 9,5, les deux compositions sont mélangées immédiatement avant l'application sur les cheveux humains, et la composition (AB) prête à l'emploi ainsi préparée a un pH entre 7 et 8.

2. Produit pour la déformation durable des cheveux humains selon la revendication 1, caractérisé en ce que la composition (A) contient un acide thioglycolique et/ou l'un de ses sels en combinaison avec des hydrochlorures d'aminoacide et au moins un polyol ou son éther de méthyle ou d'éthyle en solution aqueuse.

3. Produit selon l'une des revendications 1 ou 2, caractérisé en ce que la composition (A) a un pH entre 5,0 et 6,0.

4. Produit selon l'une des revendications 1 ou 2, caractérisé en ce que la composition (B) a un pH entre 8,1 et 8,6.

5. Produit selon l'une des revendications précédentes, caractérisé en ce que la composition (AB) prête à être appliquée sur les cheveux obtenue par mélange des compositions (A) et (B) a un pH entre 7,3 et 7,7.

6. Produit selon l'une des revendications précédentes, caractérisé en ce qu'il contient l'hydrochlorure de cystéine comme hydrochlorure d'aminoacide.

7. Produit selon l'une ou plusieurs des revendications précédentes, caractérisé en ce qu'il contient l'hydrochlorure de glycine comme chlorure d'aminoacide.

8. Produit selon l'une ou plusieurs des revendications précédentes, caractérisé en ce que la composition (A) contient de 1 à 15 % en poids d'un polyol ou de son éther de méthyle ou d'éthyle.

9. Produit selon la revendication 8, caractérisé en ce que la composition (A) contient du 1,2-propanediol et/ou de la glycérine.

10. Produit selon la revendication 8, caractérisé en ce que la composition (A) contient du 1-méthoxypropanol(-2) et/ou le monométhyléther de dipropylène glycol.

11. Produit selon l'une ou plusieurs des revendications précédentes, caractérisé en ce qu'il contient de 1 à 5 % en poids d'hydrogénocarbonate d'ammonium.
